# EUROPEAN PATENT SPECIFICATION

(11) **EP 4 033 391 B1**
(45) Date of publication and mention of the grant of the patent: **02.12.2026**
(21) Application number: 20864840.2
(22) Date of filing: 19.08.2020
(51) Int. Cl.: G16C 20/30, G06F 30/10, G06F 30/27

(54) **MATERIAL PROPERTY PREDICTION SYSTEM AND MATERIAL PROPERTY PREDICTION METHOD**
SYSTEM ZUR VORHERSAGE VON MATERIALEIGENSCHAFTEN UND VERFAHREN ZUR VORHERSAGE VON MATERIALEIGENSCHAFTEN
SYSTÈME DE PRÉVISION DE PROPRIÉTÉS DE MATÉRIAU ET PROCÉDÉ DE PRÉVISION DE PROPRIÉTÉS DE MATÉRIAU

(30) Priority: 18.09.2019 JP 2019169651
(43) Date of publication of application: 27.07.2022
(73) Proprietor: Hitachi, Ltd., Tokyo 100-8280 (JP)
(72) Inventor: ASAHARA Akinori, Tokyo 100-8280 (JP); HAYASHI Takayuki, Tokyo 100-8280 (JP); KANAZAWA Takuya, Tokyo 100-8280 (JP); MORITA Hidekazu, Tokyo 100-8280 (JP)
(74) Representative: Mewburn Ellis LLP
(86) International application number: PCT/JP2020/031267
(87) International publication number: WO 2021/054026

(56) References cited:
- WO-A1-2019/172280
- JP-A- 2010 277 328
- JP-A- 2017 091 526
- JP-A- 2019 082 790
- JP-A- 2019 086 817
- US-B2- 8 311 955
- VARNEK ALEXANDRE ET AL: "Inductive Transfer of Knowledge: Application of Multi-Task Learning and Feature Net Approaches to Model Tissue-Air Partition Coefficients", JOURNAL OF CHEMICAL INFORMATION AND MODELING, vol. 49, no. 1, 6 January 2009 (2009-01-06), US, pages 133 - 144, XP093079742, ISSN: 1549-9596, DOI: 10.1021/ci8002914

## Description

### Technical Field

The present invention relates to a technology that supports experiments in materials science among others.

### Background Art

Along with development of a statistical processing technology regarding data analysis, there is a rising demand for carrying out data analysis in materials science as well. Particularly, in a field of materials science, a method called screening is known in which a selection of candidates for a next experiment is made based on known data to perform development of new materials efficiently.

In Patent Literature (PTL) 1, a design support method is described in which knowledges in a nanoscale domain are linked and structured in a same concept scheme independently of material types and applied usefully to new material design independent of material types.

In PTL 2, descriptions are provided as below: through the use of quantum statistic values that are obtained through statistic processing of quantum thermodynamics state quantities specific to elements constituting a reaction system, out of substances with the same number of elements, for which the number or percentage of elements that constitute a reaction system differs, a selection is made of only those substances that have the same physical property value; by inducing multiple simultaneous liner equations that are as many as or more than the number of elements constituting each of those substances and finding a solution of the equations, it is enabled to design the material of a metallic or non-metallic substance having targeted physical and chemical properties and functionality.

As a screening method, various sorts of experimental data are input to an information system, a model is built for predicting an experiment result through machine learning, and screening is performed based on prediction through the model. For this prediction, a method that takes various parameters regarding material design as arguments and evaluates a function that returns a material property by a regression analysis is well known.

### Citation List

### Patent Literature

PTL 1: Japanese Patent Application Laid-Open No. 2003-178102
PTL 2: Japanese Patent Application Laid-Open No. 2004-086892
PTL 3: US 8 311 955 B2 discloses a bootstrap method for oil property prediction

### Non patent literature

Varnek et al (J. Chem. Inf: Model. 2009, 49, 133-144) discloses Inductive Transfer of Knowledge: Application of Multi-Task Learning and Feature Net Approaches to Model Tissue-Air Partition Coefficients

### Summary of Invention

### Technical Problem

In material development, increasing the accuracy of predicting material properties makes it possible to identify a promising potential of a candidate for a new material more exactly and, by dispensing with unnecessary experiments, it is expected that efficient material development can be conducted.

In the regression analysis, variables that correspond to arguments of a function are called explanatory variables and a value that corresponds to a return value of the function is called an objective variable. In predicting a property of a material, the material property is taken as the objective variable and explanatory variables representing the features of the material are selected so that the material property can be predicted. Increase or decrease in the accuracy of the prediction depends on how to select the explanatory variables and, therefore, it is important to prepare a variation of explanatory variables to be adaptable for prediction of a wide range of material properties.

An attempt to predict material properties using past data is disclosed in PTL 1 and PTL 2. However, a general process for material development starts development with certain compositions and a manufacturing process and, for a material found to have an effective property, further takes measures with its related composition and manufacturing process.

In fact, there is a problem in which, in an initial phase of development, only a very small amount of data can be used for a task that has just begun. When attempting to use information of past data, in most cases, data representing well-prepared material properties is only such data available for the task to address, because material properties that are targeted differ task by task. In addition, in some cases, even experiments that aim at finding like properties use different measurement methods and it is often hard to repurpose resulting data straightforwardly.

A problem that is addressed by the present invention is to provide a method that improves the accuracy of predicting material properties by making effective use of past data.

### Solution to Problem

One preferred aspect of the present invention resides in a system as set forth in claim 1.

Material composition is the structural formula of the material.

Another preferred aspect of the present invention resides in a method as set forth in claim 8. Preferred embodiments of the invention are specified in the dependent claims.

### Advantageous Effects of Invention

It is possible to improve the accuracy of predicting material properties by making effective use of past data.

### Brief Description of Drawings

Figure 1 is a functional block diagram depicting an example of an outlined configuration of an example.
Figure 2 is a block diagram depicting an example of a physical implementation configuration of the example.
Figure 3 is a conceptual diagram illustrating an example of procedures for using the example.
Figure 4 is a flowchart illustrating an example of a material DB update process in the example.
Figure 5 is an image diagram illustrating an example of a screen that is displayed for accepting experimental data in the example.
Figure 6 is a tabular diagram illustrating an example of the structure of experimental data in the example.
Figure 7 is a tabular diagram illustrating an example of an experimental data table in a material DB in the example.
Figure 8 is a conceptual diagram illustrating an example of task data.
Figure 9 is an explanatory diagram illustrating a concept of cross-task compatible feature values.
Figure 10 is a flowchart illustrating an example a material property prediction process in the example.
Figure 11 is an image diagram illustrating an example of a material property prediction display in the example.
Figure 12 is a tabular diagram illustrating an example of the structure of data for predicting material properties in the example.

### Description of Embodiments

An embodiment is now described in detail with the aid of the drawings. However, the present invention should not be construed to be limited to the following description of the embodiment. The invention is defined in the appended claims.

In a configuration of the invention which will be described hereinafter, for identical parts or parts having like functions, identical reference numerals are used in common across different drawings and duplicated description of those parts may be omitted.

Multiple elements having the same or like functions, if any, may be assigned the same reference numeral with different subscripts and described. However, when it is not necessary to individualize those multiple elements, the subscripts may be omitted in describing them.

Notation of "first", "second", "third", etc. herein is prefixed to identify components, but it is not necessarily intended to confine the components to a certain number, sequence, or contents. In addition, numbers to identify components are used on a per-context basis; a number used in one context does not always denote the same component in another context. Additionally, it is not precluded that a component identified by a number also functions as a component identified by another number.

In some cases, the position, size, shape, range, etc. of each component depicted in a drawing or the like may not represent its actual position, size, shape, range, etc. with the intention to facilitate understanding of the invention. Hence, the present invention is not necessarily to be limited to a position, size, shape, range, etc. disclosed in a drawing or the like.

### <Example 1>

### <1. System configuration>

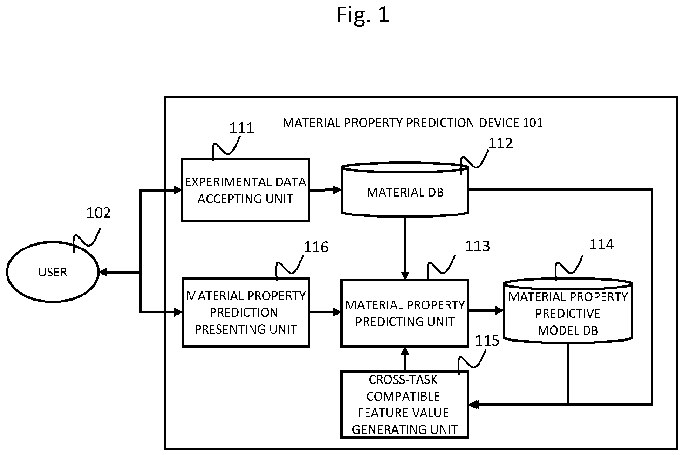

Figure 1 depicts an example of a material property prediction device of Example 1. The material property prediction device (101) of the present example is a device that accepts operation by a user (102) and includes an experimental data accepting unit (111) that receives experimental data from the user and a material database (DB: Data Base) (112) on a per-task basis in which the features and properties of materials are stored. Here, a task means a set of data that a user can define freely; e.g., data obtained from an experiment and a development and assumed to be created by different persons and for different purposes.

The material property prediction device (101) also includes a material property predicting unit (113) that generates a material property predictive model to predict material properties and predicts unmeasured material properties using a material property predictive model and a material property predictive model DB (114) to store material property predictive models.

The material property predicting unit (113) generates a material property predictive model by using feature values obtained from data of measured values of a material property from the material DB (112) and feature values obtained from a cross-task compatible feature value generating unit (115) and predicts an unknown property. The cross-task compatible feature value generating unit (115) generates new feature values from data in the material DB (112) and the material property predictive model DB (114). A material property prediction presenting unit (116) presents a result of a prediction made by the material property predicting unit (113) to the user (102).

In the present example, the material property prediction device (101) was assumed to be configured as an information processing device like a server including an input device, an output device, a storage device, and a processing device. Computation and control functions among others are implemented by carrying out a defined process in cooperation with other elements of hardware in such a manner that a program stored in the storage device is executed by the processing device. Figure 1 illustrates functional blocks instead of the hardware configuration of an information processing device. As the respective functional blocks, programs to be executed by a computer or the like, their functions or means for implementing the functions may be referred to as "functions", "means", "sections", "units", "modules", etc.

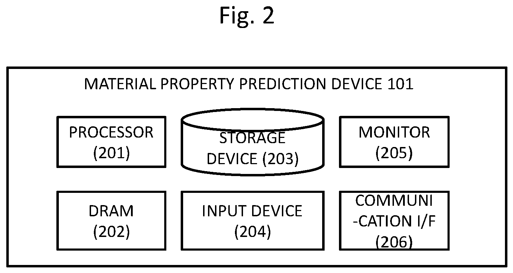

Figure 2 depicts an example of a physical implementation configuration of Example 1. The material property prediction device (101) can be implemented by using a commonly used computer; that is, a device including a processor (201) having computational performance, a DRAM (Dynamic Random Access Memory) (202) which is a volatile and temporary memory with areas readable and writable at high speed, a storage device (203) that provides for permanent storage areas using a HDD (hard disk device), a flash memory, etc., an input device (204) which is a mouse and a keyboard, etc. for user operation, a monitor (205) for presenting an operation to the user, and an interface (206) such as a serial port for communication with an external entity.

In Figure 1, the experimental data accepting unit (111), the material property predicting unit (113), the cross-task compatible feature value generating unit (115), and the material property prediction presenting unit (116) can be implemented in such a manner that the processor (201) executes programs recorded in the storage device (203). The material DB (112) and the material property predictive model DB (114) can be implemented in such a manner that the processor (201) executes a program to store data into the storage device (203).

The configuration of Figure 2 may be configured on a single computer or any part thereof may be configured on another computer connected via a network. In other words, the same system as discussed herein may be configured with a plurality of computers.

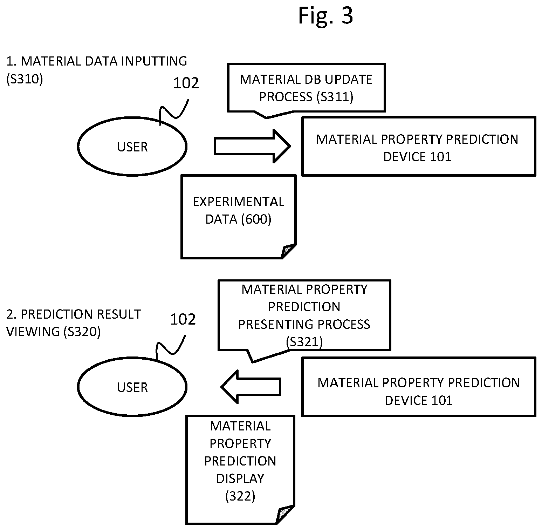

Figure 3 schematically illustrates procedures for using the system of Example 1. Example 1 enables the execution of two procedures as follows: material data inputting (S310), i.e., a user inputs data concerned in predicting material properties; and prediction result viewing (S310) to check a result of predicted material properties.

Material data inputting (S310) is a procedure of inputting experimental data (600) which is a data set in which data of a material for which an experiment was conducted and data of a material for which an experiment is going to be conducted have been stored to the material property prediction device (101). In response to this data, the material property prediction device executes a material DB update process (S311), thereby updating internally stored information.

In the prediction result viewing (S320), the material property prediction device executes a material property prediction presenting process (S321) in response to a request of the user (102) and presents a material property prediction display (322) which is a screen in which a result of predicted material properties is visualized.

### <2. Material data inputting process>

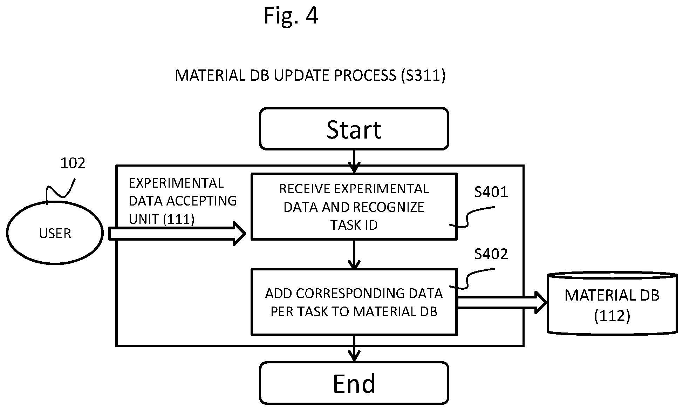

Figure 4 illustrates an example of a processing procedure of the material DB update process (S311). In the material DB update process (S311), the experimental data accepting unit (111) first receives experimental data (600) from the user (102) and recognizes or adds a task ID (S401). Then, the process updates or adds the corresponding data per task to the material DB (112) (S402).

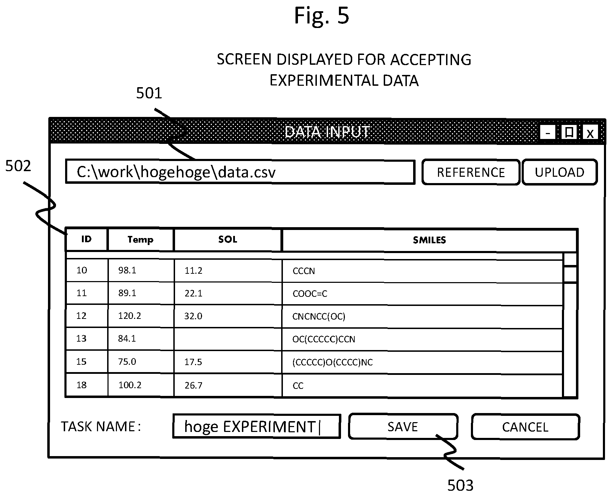

Figure 5 illustrates an example of a screen that is displayed on the monitor (205) for receiving experimental data (600) from the user (102) in the first step (S401) of the material DB update process (311). In Example 1, the user (102) pre-stores experimental data in a file and specifies the file location in a text box (501); in this way, the user passes experimental data (600). In the file that is passed, tabular data is described in a CSV (Comma Separated Value) format which is publicly known and its interpreted result rendered in a tabular form is displayed in a table screen (502).

In Figure 5, illustrated is a table with the following columns: "ID" which is the identifier of an experiment whose information is described; "Temp" that indicates temperature when the experiment was conducted; "SOL" that indicates water solubility measured at that time; and "SMILES" which is a string representing the structural formula of a material. Water solubility is a material property that an experimenter wants to predict in this example and blank data in the SOL column indicates a nonexperimental condition. Note that this way of passing data to the device is exemplary and another method may be applicable using any format in which, as information convertible to a tabular form, experimental data including structural formulas of materials and a material property can be passed to the device. Information is displayed in the table screen (502) and saved in the material DB (112) using a button (503).

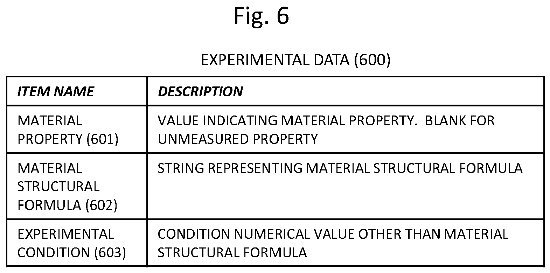

Figure 6 illustrates an example of the structure of one record of the experimental data (600). One record is created for one material having a particular composition and obtained in a manufacturing process. In the present example, the experimental data (600) is information in which one record includes the following pieces of information: material property (601), material structural formula (602) which is information that can express the material structural formula, such as, e.g., in the SMILES format, and experimental condition (603) indicating a condition when the experiment was conducted, such as temperature and pressure. The experimental data (600) is a collection of one or more such records. These pieces of information correspond to the respective column items of the table screen (502) in Figure 5. In the present example, correspondence between each item and which element is determined by correspondence to a predetermined item name. The user (102) may be prompted to input this correspondence relationship from the screen. Moreover, as for the material property (601), a value revealed by the experiment or the like is stored or a blank is stored if it is nonexperimental. Other information such as a task name may be added to the experimental data (600).

The first step (S401) of the material DB update process (S311) of Figure 4 interprets and formats the experimental data (600) and stores it as an experimental data table in the material DB (112).

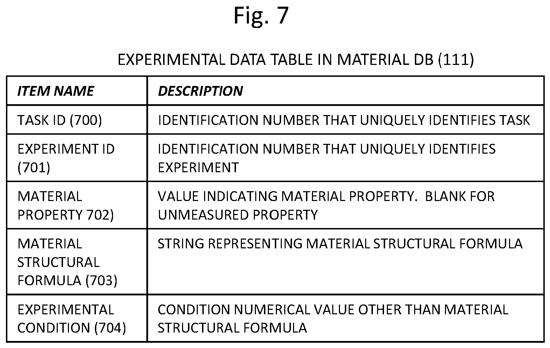

Figure 7 illustrates information in one record of an experimental data table. This data includes experiment ID (701) assigned to each experiment in a serial numbering scheme or the like so that the experiment can be identified uniquely, material property (702) derived from the material property (601) of the experimental data (600), material structural formula (703) derived from the material structural formula (602) of the experimental data (600), and experimental condition (704) derived from the experimental condition (603). Information from which these items of data are derived may be converted in units and formats and transformed into a coherent representation.

A task ID (700) is an identification number that uniquely identifies a task. In Example 1, it is assumed to handle one file as one task and, therefore, a task ID corresponds to a filename of a real data file. A task ID (700) should be added in a serial numbering scheme when registering in the material DB (112). If correspondence between a file and a task is not fixed, its registration may be made in the following manner: when registering in the material DB (112), a question that "a file you are going to upload now corresponds to what task?" is presented to the user to ask the user to input the correspondence. The format of the experimental data table is required to be the same for registered data and added data. The user can define the material property (702) and the experimental condition (704) optionally and also can set the number of material properties and experimental conditions freely.

### <3. Cross-task compatible feature values>

A feature of the present example is improving the accuracy of predicting material properties by using data of existing tasks even in a situation where there are few data pieces. In an initial phase of a material development process, the amount of available data is very small. Before explaining a concrete example, a concept of the present example is described.

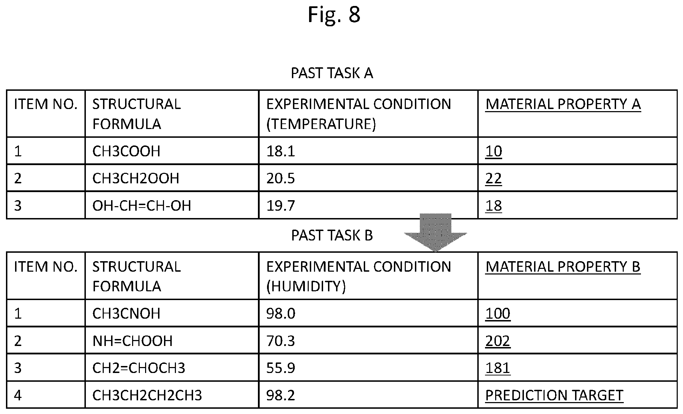

Figure 8 illustrates an example of task data that is stored in the material database (112) on a per-task basis. As illustrated in Figure 8, when attempting to use information of any other task, in most cases, data representing well-prepared material properties is only such data available for the task to address, because material properties that are targeted usually differ task by task. In addition, in some cases, even experiments that aim at finding like properties use different measurement methods and it is often hard to repurpose resulting data straightforwardly.

In the example of Figure 8, a past task A and a past task B have data under different experimental conditions, temperature, and humidity, and for different material properties, A and B; therefore, their data cannot be used interchangeably for property prediction as it is. In the present example, it is enabled to increase the number of explanatory variables by using past task data as "information for creating feature values". Here, feature values that are newly created are referred to as "cross-task compatible feature values".

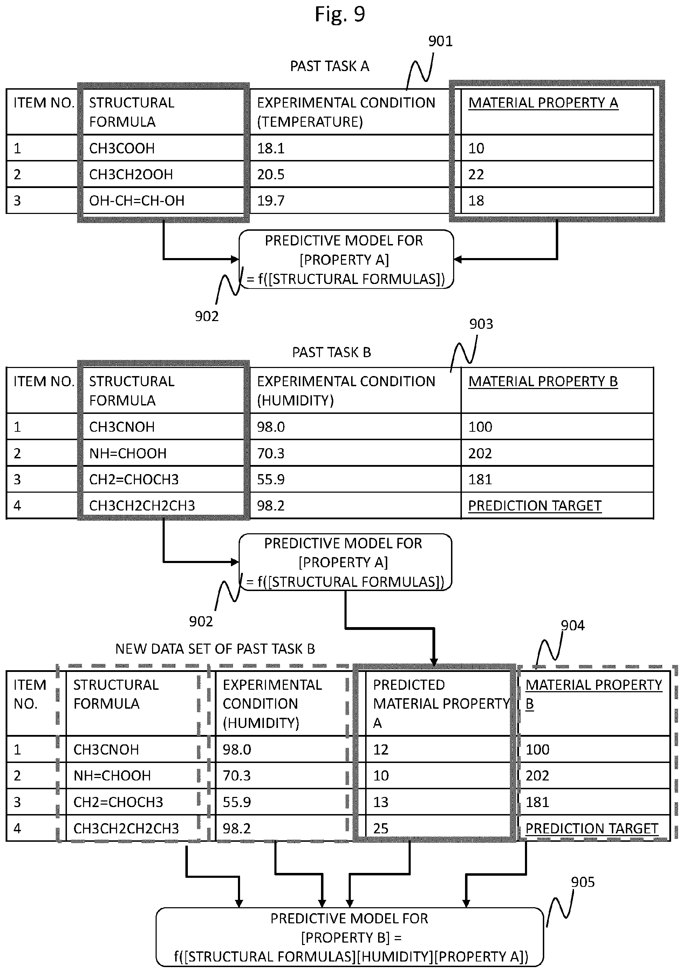

A process that uses information about past tasks as "information for creating feature values" is described with Figure 9. Using data of the past task A (901), the process first generates (learns) a predictive model (902) to predict a material property A from structural formulas, assuming the objective variable as the material property A of a known material and the explanatory variables as the structural formulas. This model can be generated through supervised machine leaning which is known, by using, e.g., regression trees, random forests, support vector regression, Gaussian process regression, neural networks, etc.

The process then predicts the material property A by applying the structural formulas in the data of the past task B (903) to the predictive model (902). The process adds the material property A to the data of the past task B, thus generating a new data set (904). If the same structural formula as in the past task B is included in the past task A, its material property in the past task A may be added as is to the new data set. This material property A corresponds to cross-task compatible feature values.

Upon having obtained the new data set (904), the process generates a predictive model (905) to predict a material property B, taking known data of the material property B (item Nos. 1, 2, and 3) in the data set as teacher data. At this time, the explanatory variables are the structural formulas, experimental condition (humidity), and the material property A and the objective variable is the material property B. The predictive model (905) can be generated through supervised machine leaning which is known.

The process inputs data (item No. 4) for which the material property B should be predicted to the generated predictive model (905) and obtains the material property B. By adding the material property A as new feature values (cross-task compatible feature values), it can be expected to improve the prediction accuracy in comparison with when the past task B data is used as it is. This is considered as effective particularly when there is a correlation between the material properties A and B.

With the understanding of the concept discussed above, a flow of a concrete process for prediction result viewing is described.

### <4. Process for prediction result viewing>

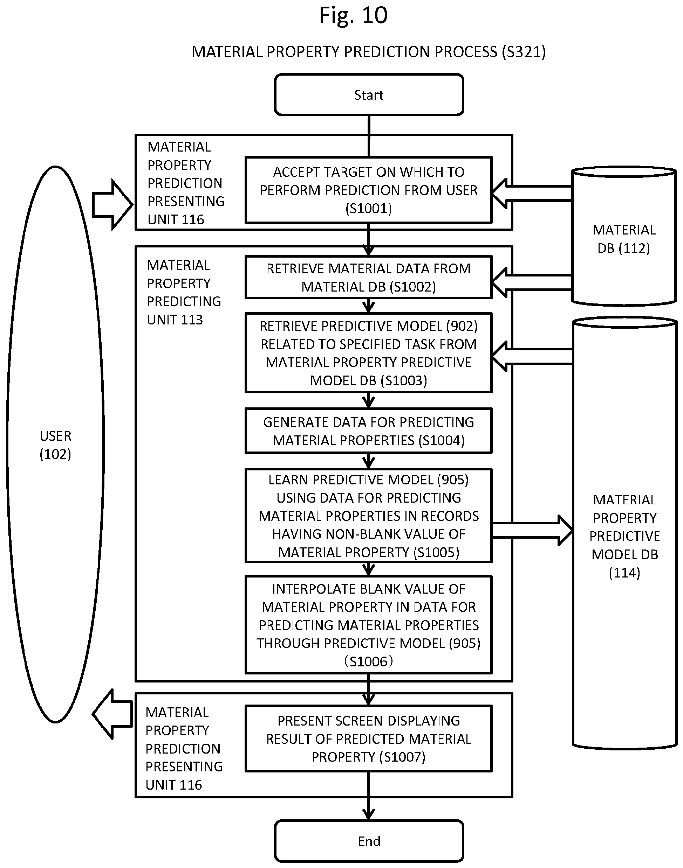

The material property prediction presenting process (S321) for prediction result viewing (S320) is described with Figure 10. In the following description, a description in relation to the concept illustrated in Figure 9 is also provided with a reference numeral in a series of 901 to 905 in Figure 9.

First, the material property prediction presenting unit (116) presents the material property prediction display (322) to the user (102) and receives the specification of an experimental data table as a target of property prediction (S1001). At this time, a task ID is used to specify the designation of an experimental data table stored in the material DB (112). Here, it is assumed that experimental data has already been stored in the material DB (112).

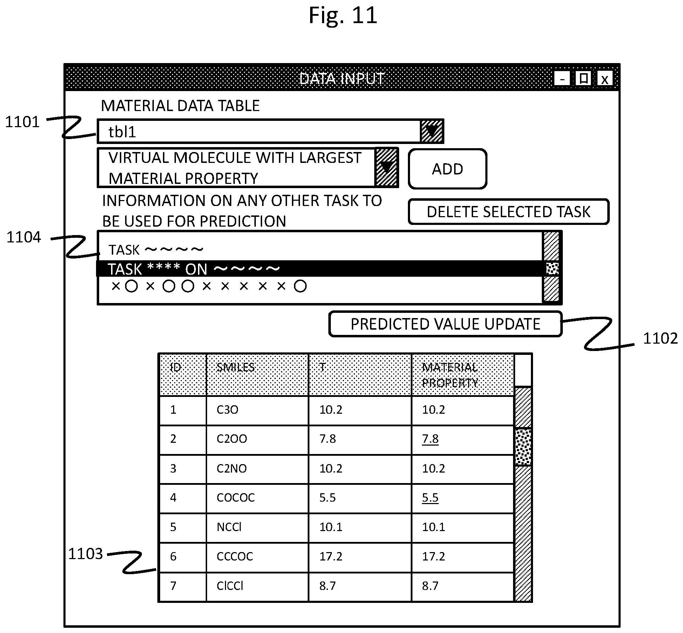

Figure 11 illustrates an example of a screen displayed on the monitor (205) for accepting a request from the user (102) and a screen for the material property prediction display (322) in which a result of predicted material properties is visualized.

In a drop-down box (1101) in the figure, the designation of an experimental data table is displayed as a candidate. When the user specifies a task ID and presses the predicted value update button (1102), the material property prediction presenting unit (116) sends a command to execute interpolation by a predicted value for blank data of material property (702) in the records of the experimental data table (Figure 7) to the material property predicting unit (113), and a result is to be displayed in the screen (1103). Underlined values of the material property in Figure 11 are those obtained by the interpolation of blank data.

Upon receiving the above command to execute interpolation from the material property prediction presenting unit (116), the material property predicting unit (113) retrieves the data of the experimental data table specified by the task ID (700) from the material DB (112) (S1002). Also, in the screen (1104) in Figure 11, any other task is selected that is used to generate cross-task compatible feature values. The material property predicting unit (113) retrieves a predictive model (902) related to the selected other task from the material property predictive model DB (114) (S1003).

Data retrieved in the processing step (S1002) as described with the flowchart of Figure 10 corresponds to the data of the past task B (903) in Figure 9. The predictive model related to the task retrieved in the processing step (S1003) corresponds to the predictive model (902) generated from the data of the past task A (901) in Figure 9.

In the above description, it is assumed that the predictive model (902) has already been created and is called by the task ID (700) from the material property predictive model DB (114). If the corresponding predictive model (902) does not exist in the material property predictive model DB (114), learning and creating the predictive model (902) should be executed, assuming the material structural formulas in the data of the past task A as the explanatory variables and the material property of a known material as the objective variable, as illustrated in Figure 9.

Then, the material property predicting unit (113) generates data for predicting material properties (S1004). This processing corresponds to predicting the material property A by applying the structural formulas in the data of the past task B (903) to the predictive model (902) and adding the material property A to the data of the past task B, thus generating a new data set (904). At this time, the cross-task compatible feature value generating unit (115) executes prediction of the material property A (cross-task compatible feature values) by using the predictive model (902) retrieved in the pressing step (S1003).

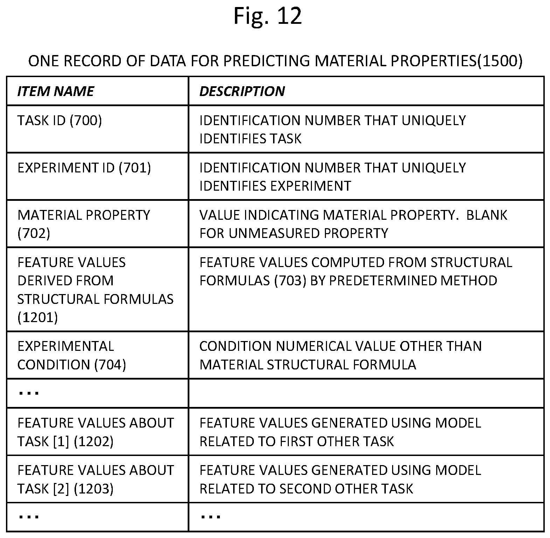

Figure 12 illustrates the structure of one record (1500) of the data for predicting material properties. The contents of one record take over the task ID (700), experiment ID (701), material property (701), and experimental condition (704) in the experimental data table (Figure 7) of the data of the past task B (903). The record also includes feature values derived from structural formulas (1201). The feature values derived from structural formulas are computed from the material structural formulas (703). As a method for computing feature values from structural formulas, there is a publicly known method such as fingerprinting.

The data for predicting material properties includes feature values (1202, 1203) created through the predictive model (902) related to any other task, i.e., cross-task compatible feature values. The description with regard to Figure 9 assumes that any other task is another one, the past task A, and cross-task compatible feature values are of one material property A to be predicted. However, feature values created through the predictive model (902) related to any other task may be those of one material property or any number of material properties. Also, multiple other tasks may be used.

From the data for predicting material properties except for records in which material property (702) is unmeasured, i.e., blank, the material property predicting unit (113) assigns items excepting task ID (700), experiment ID (701), and material property (702) to the explanatory variables and the material property (702) to the objective variable, executes a regression analysis which is publicly known, obtains a prediction function, and learns a predictive model (905) (S1005). The created predictive model (905) is stored into the material property predictive model DB (114) together with the task ID of the data from which the predictive model (905) was generated.

Given that the prediction function is written as y = f (x1, x2, ...), where y is the objective variable and x1, x2, ... are the explanatory variables, this procedure means defining the function form of f, i.e., defining x1, x2, ... so that y can be predicted. In the case of the present example, supposing the use of the data for predicting material properties in Figure 12, the predictive model (905) is generated through learning of a regression analysis on the function: [material property (702)] = f ([feature values derived from structural formulas (1201)], [experimental condition (704)], [feature values about task [1] (1202)], [feature values about task [2] (1203)] ....).

This learning corresponds to generating the predictive model (905) in the bottom row of Figure 9. Although experimental condition (704) is one type that is humidity in Figure 9, there may be any number and any type of experimental conditions, provided that relevant data exists. As experimental conditions, there are, e.g., material manufacturing conditions; however, they may be omissible if there is no relevant data. Also, as noted previously, although cross-task compatible feature values are of one material property A to be predicted, as illustrated in Figure 9, there may be a plurality of items of such values, as in the formula provided above.

Algorithms for the regression analysis may be those that are publicly known; regression trees, LASSO, random forests, support vector regression, Gaussian process regression, neural networks, etc. can be used. Note that an increase in the number of explanatory variables is made in the present example and regression trees, and random forests are suitable for increasing the number of explanatory variables rather than support vector regression. Particularly, with nonlinear random forests, prediction at high accuracy can be expected.

After thus generating the predictive model (905), the material property predicting unit (113) selects a record in which material property (702) is unmeasured, i.e., blank and computes a predicted value of the material property (702) using the foregoing prediction function y = f (x1, x2, ...) (S1006).

The computed predictive value is displayed by the material property prediction presenting unit (116) in the screen on the monitor (205), as illustrated in Figure 11 (S1007). Note that, although feature values of spatial structures and experimental conditions are only used as explanatory variables in the present example, in fact, any other amount (e.g., molecular weight or charge) may be derived, added, and used.

Although structural formulas are used when creating feature values about any other task in the example discussed hereinbefore, data of composition and others may be used as long as the data is common across tasks data. Additionally, a method in which prediction can be made using structural formulas as such is also publicly known and the scheme is the same in that case as well.

According to the example described hereinbefore, using data stored when material properties were predicted in any other past task, a model is created that is compatible with a prediction that is executed currently and the accuracy is improved by increasing the number of explanatory variables through the model. Although, e.g., a task (the past task B in Figure 9) in the beginning of research and development has few data pieces, the present example enables it to make good use of data of a past task (the past task A in Figure 9) for which, e.g., research and development are complete and the amount of data is large. This can overcome a problem in which, when prediction of material properties is performed, its accuracy is low due to a small amount of data. Accordingly, more accurate prediction can be performed in the phase of prediction and evaluation for screening in an experiment plan. In consequence, experiment plans will become easy to make and, furthermore, a good material can be developed with a reduced number of times experiments are performed. For example, it is possible to find out a parameter that is predicted to improve a property and do work, prioritizing the experimental condition of the parameter.

### Reference Signs List

101: material property prediction device
102: user
111: experimental data accepting unit
112: material DB
113: material property predicting unit
114: material property predictive model DB
115: cross-task compatible feature value generating unit
116: material property prediction presenting unit

## Claims

1. A material property prediction system that is a system to carry out prediction of material properties by processing task data including a plurality of records, each including a material composition being a structural formula of the material, an experimental condition indicating a condition when an experiment associated with a material property was conducted, and the material property, the system comprising a material property prediction presenting unit (116), a cross-task compatible feature value generating unit (115), and a material property predicting unit (113),
wherein the material property prediction presenting unit (116) accepts a specification of first task data that includes a record in which a material property is unknown and is to be a target of material property prediction through a first predictive model;
wherein the cross-task compatible feature value generating unit (115) generates a second predictive model by using material compositions and a known second material property in second task data different from the first task data;
wherein the cross-task compatible feature value generating unit (115) predicts feature values from material compositions in the first task data based on a material property that is defined in the second data from material compositions in the first task data by using the second predictive model;
wherein the material property predicting unit (113) generates the first predictive model by using the material compositions, the experimental conditions, the feature values, and the known material property in the first task data; and
wherein the material property predicting unit (113) inputs the material composition, the experimental condition, and the feature value in a record in which the material property is unknown in the first task data to the first predictive model and predicts the unknown material property.

2. The material property prediction system according to claim 1,
wherein the task data can be retrieved from a material database (112);
the material database (112) stores a plurality of tasks data pieces and data on the experimental condition and the material property includes data in which different conditions and properties are defined across the tasks data pieces;
the material property prediction presenting unit (116) accepts a specification of second task data different from the first task data;
the cross-task compatible feature value generating unit (115) retrieves the second task data from the material database to generate the second predictive model.

3. The material property prediction system according to claim 2, including the material database (112) in which the following are stored:
the first task data including a plurality of records, each including a material composition, a first experimental condition, and a first material property; and
the second task data including a plurality of records, each storing a material composition and a second experimental condition defined different from the first experimental condition.

4. The material property prediction system according to claim 2, including the material database (112) in which the following are stored:
the first task data including a plurality of records, each including a material composition, a first experimental condition, and a first material property; and
the second task data including a plurality of records, each storing a material composition and a second material property defined different from the first material property.

5. The material property prediction system according to claim 2, provided with a material property predictive model database (114) storing at least one of the first predictive model and the second predictive model.

6. The material property prediction system according to claim 5, wherein the second predictive model is managed in relation to the second task data.

7. The material property prediction system according to claim 1, wherein the first predictive model is configured using a random forest.

8. A material property prediction method that is a method for predicting material properties by an information processing device including an input device, a storage device, and a processor,
wherein, when generating a first predictive model for predicting a first material property from first task data, the first task data including a plurality of records, each including a material composition being a structural formula of the material, an experimental condition indicating a condition when an experiment associated with the first material property was conducted, and the first material property, the method executes:
a first step of preparing a second predictive model that is to predict a second material property defined different from the first material property by using material compositions and the known second material property in second task data different from the first task data;
a second step of predicting the second material property by applying the first data to the second predictive model;
a third step of generating the first predictive model, taking the structural formula of the material as a first explanatory variable, the second material property as a second explanatory variable, the experimental condition as a third explanatory variable, and the first material property as an objective variable, and
a fourth step of predicting the first material property for a record in which the first material property is unknown by using the first predictive model and the first data.

9. The material property prediction method according to claim 8, using a material database on a per-task basis,
wherein first task data regarding a first task and second task data regarding a second task are stored in the material database.

10. The material property prediction method according to claim 9, wherein the first task data further includes first information about material manufacturing conditions.

11. The material property prediction method according to claim 10, wherein the second task data further includes second information defined different from the first information about material manufacturing conditions.

12. The material property prediction method according to claim 8, wherein a random forest is used as the first predictive model.

## Patentansprüche

1. Materialeigenschaftsvorhersagesystem, das ein System zum Durchführen einer Vorhersage über Materialeigenschaften durch Verarbeiten von Aufgabendaten, die eine Vielzahl von Einträgen umfassen, ist, jeweils umfassend eine Materialzusammensetzung, die eine Strukturformel des Materials ist, eine Versuchsbedingung, die eine Bedingung beim Durchführen eines mit einer Materialeigenschaft zusammenhängenden Versuchs angibt, und die Materialeigenschaft, wobei das System eine Materialeigenschaftsvorhersagepräsentationseinheit (116), eine aufgabenübergreifend kompatible Merkmalswert-Erzeugungseinheit (115) und eine Materialeigenschaftsvorhersageeinheit (113) umfasst,
wobei die Materialeigenschaftsvorhersagepräsentationseinheit (116) über ein erstes Vorhersagemodell eine Spezifizierung von ersten Aufgabendaten, welche einen Eintrag umfassen, in dem eine Materialeigenschaft unbekannt ist und ein Ziel einer Materialeigenschaftsvorhersage ist, annimmt;
wobei die aufgabenübergreifend kompatible Merkmalswert-Erzeugungseinheit (115) ein zweites Vorhersagemodell erzeugt, indem sie Materialzusammensetzungen und eine bekannte zweite Materialeigenschaft in zweiten Aufgabendaten, die sich von den ersten Aufgabendaten unterscheiden, verwendet;
wobei die aufgabenübergreifend kompatible Merkmalswert-Erzeugungseinheit (115) Merkmalswerte aus Materialzusammensetzungen in den ersten Aufgabendaten basierend auf einer Materialeigenschaft, die in den zweiten Daten aus Materialzusammensetzungen in den ersten Aufgabendaten definiert ist, unter Verwendung des zweiten Vorhersagemodells vorhersagt;
wobei die Materialeigenschaftsvorhersageeinheit (113) das erste Vorhersagemodell unter Verwendung der Materialzusammensetzungen, der Versuchszustände, der Merkmalswerte und der bekannten Materialeigenschaft in den ersten Aufgabendaten erzeugt; und
wobei die Materialeigenschaftsvorhersageeinheit (113) die Materialzusammensetzung, die Versuchsbedingung und den Merkmalswert in einem Eintrag, in dem die Materialeigenschaft in den ersten Aufgabendaten unbekannt ist, in das erste Vorhersagemodell eingibt und die unbekannte Materialeigenschaft vorhersagt.

2. Materialeigenschaftsvorhersagesystem nach Anspruch 1,
wobei die Aufgabendaten aus einer Materialdatenbank (112) abgerufen werden können; wobei
die Materialdatenbank (112) eine Vielzahl von Aufgabendatenelementen und Daten über die Versuchsbedingung speichert und die Materialeigenschaft Daten umfasst, in denen verschiedene Zustände und Eigenschaften quer durch die Aufgabendatenelemente definiert sind; wobei
die Materialeigenschaftsvorhersagepräsentationseinheit (116) eine Spezifizierung von zweiten Aufgabendaten, die sich von den ersten Aufgabendaten unterscheiden, annimmt; wobei
die aufgabenübergreifend kompatible Merkmalswert-Erzeugungseinheit (115) die zweiten Aufgabendaten aus der Materialdatenbank abruft, um das zweite Vorhersagemodell zu erzeugen.

3. Materialeigenschaftsvorhersagesystem nach Anspruch 2, umfassend die Materialdatenbank (112), in der Folgendes gespeichert ist:
die ersten Aufgabendaten, umfassend eine Vielzahl von Einträgen, die jeweils eine Materialzusammensetzung, eine erste Versuchsbedingung und eine erste Materialeigenschaft umfassen; und
die zweiten Aufgabedaten, umfassend eine Vielzahl von Einträgen, die jeweils eine Materialzusammensetzung und eine zweite Versuchsbedingung, welche anders definiert ist als die erste Versuchsbedingung, speichern.

4. Materialeigenschaftsvorhersagesystem nach Anspruch 2, umfassend die Materialdatenbank (112), in der Folgendes gespeichert ist:
die ersten Aufgabendaten, umfassend eine Vielzahl von Einträgen, die jeweils eine Materialzusammensetzung, eine erste Versuchsbedingung und eine erste Materialeigenschaft umfassen; und
die zweiten Aufgabendaten, umfassend eine Vielzahl von Einträgen, die jeweils eine Materialzusammensetzung und eine zweite Materialeigenschaft, welche anders definiert ist als die erste Materialeigenschaft, speichern.

5. Materialeigenschaftsvorhersagesystem nach Anspruch 2, das mit einer Materialeigenschaftsvorhersagemodelldatenbank (114) bereitgestellt ist, die zumindest eines aus dem ersten Vorhersagemodell und dem zweiten Vorhersagemodell speichert.

6. Materialeigenschaftsvorhersagesystem nach Anspruch 5, wobei das zweite Vorhersagemodell in Bezug auf die zweiten Aufgabendaten verwaltet wird.

7. Materialeigenschaftsvorhersagesystem nach Anspruch 1, wobei das erste Vorhersagemodell unter Verwendung eines Random-Forest konfiguriert ist.

8. Materialeigenschaftsvorhersageverfahren, das ein Verfahren zum Vorhersagen von Materialeigenschaften durch eine Informationsverarbeitungsvorrichtung ist, welche eine Eingabevorrichtung, eine Speichervorrichtung und einen Prozessor umfasst,
wobei beim Erzeugen eines ersten Vorhersagemodells zum Vorhersagen einer ersten Materialeigenschaft aus ersten Aufgabendaten die ersten Aufgabendaten eine Vielzahl von Einträgen umfassen, jeweils umfassend eine Materialzusammensetzung, die eine Strukturformel des Materials ist, eine Versuchsbedingung, die eine Bedingung beim Durchführen eines der ersten Materialeigenschaft zugeordneten Versuchs angibt, und die erste Materialeigenschaft, wobei das Verfahren Folgendes ausführt:
einen ersten Schritt des Erstellens eines zweiten Vorhersagemodells, das eine zweite Materialeigenschaft, die anders definiert ist als die erste Materialeigenschaft, vorhersagen soll, indem Materialzusammensetzungen und die bekannte zweite Materialeigenschaft in zweiten Aufgabendaten, die sich von den ersten Aufgabendaten unterscheiden, verwendet werden;
einen zweiten Schritt des Vorhersagens der zweiten Materialeigenschaft, indem die ersten Daten auf das zweite Vorhersagemodell angewandt werden;
einen dritten Schritt des Erzeugens des ersten Vorhersagemodells unter Heranziehung der Strukturformel des Materials als eine erste Erklärungsvariable, der zweiten Materialeigenschaft als eine zweite Erklärungsvariable, der Versuchsbedingung als eine dritte Erklärungsvariable und der ersten Materialeigenschaft als eine objektive Variable und
einen vierten Schritt des Vorhersagens der ersten Materialeigenschaft für einen Eintrag, in dem die erste Materialeigenschaft unbekannt ist, indem das erste Vorhersagemodell und die ersten Daten verwendet werden.

9. Materialeigenschaftsvorhersageverfahren nach Anspruch 8 unter Verwendung einer Materialdatenbank auf einer aufgabenbezogenen Basis,
wobei erste Aufgabendaten, die eine erste Aufgabe betreffen, und zweite Aufgabendaten, die eine zweite Aufgabe betreffen, in der Materialdatenbank gespeichert werden.

10. Materialeigenschaftsvorhersageverfahren nach Anspruch 9, wobei die ersten Aufgabendaten ferner erste Informationen über Materialherstellungsbedingungen umfassen.

11. Materialeigenschaftsvorhersageverfahren nach Anspruch 10, wobei die zweiten Aufgabendaten ferner zweite Informationen umfassen, die anders definiert sind als die ersten Informationen über Materialherstellungsbedingungen.

12. Materialeigenschaftsvorhersageverfahren nach Anspruch 8, wobei ein Random-Forest als das erste Vorhersagemodell verwendet wird.

## Revendications

1. Système de prédictions de propriété de matériau qui est un système pour effectuer une prédiction de propriétés de matériau par traitement de données de tâche incluant une pluralité d'enregistrements, chacun incluant une composition de matériau étant une formule structurelle du matériau, une condition expérimentale indiquant une condition lorsqu'une expérience associée à une propriété de matériau a été menée, et la propriété de matériau, le système comprenant une unité de présentation de prédiction de propriété de matériau (116), une unité de génération de valeur de caractéristique compatible entre tâches (115) et une unité de prédiction de propriété de matériau (113),
dans lequel l'unité de présentation de prédiction de propriété de matériau (116) accepte une spécification de premières données de tâche qui inclut un enregistrement dans lequel une propriété de matériau est inconnue et doit être une cible de prédiction de propriété de matériau à travers un premier modèle prédictif ;
dans lequel l'unité de génération de valeur de caractéristique compatible entre tâches (115) génère un second modèle prédictif en utilisant des compositions de matériau et une seconde propriété de matériau connue dans des secondes données de tâche différentes des premières données de tâche ;
dans lequel l'unité de génération de valeur de caractéristique compatible entre tâches (115) prédit des valeurs de caractéristique à partir des compositions de matériau dans les premières données de tâche sur la base d'une propriété de matériau qui est définie dans les secondes données à partir de compositions de matériau dans les premières données de tâche en utilisant le second modèle prédictif ;
dans lequel l'unité de prédiction de propriété de matériau (113) génère le premier modèle prédictif en utilisant les compositions de matériau, les conditions expérimentales, les valeurs de caractéristique et les propriétés de matériau connues dans les premières données de tâche ; et
dans lequel l'unité de prédiction de propriété de matériau (113) entre la composition de matériau, la condition expérimentale et la valeur de caractéristique d'un enregistrement dans lequel la propriété de matériau est inconnue dans les premières données de tâche, au premier modèle prédictif et prédit la propriété de matériau inconnue.

2. Système de prédiction de propriété de matériau selon la revendication 1,
dans lequel les données de tâche peuvent être extraites à partir d'une base de données de matériau (112) ;
la base de données de matériau (112) stocke une pluralité d'éléments de données de tâches et de données sur la condition expérimentale et la propriété de matériau inclut des données dans lesquelles différentes conditions et propriétés sont définies à travers les éléments de données de tâches ;
l'unité de présentation de prédiction de propriété de matériau (116) accepte une spécification de secondes données de tâche différentes des premières données de tâche ;
l'unité de génération de valeur de caractéristique compatible entre tâches (115) récupère les secondes données de tâche à partir de la base de données de matériau pour générer le second modèle prédictif.

3. Système de prédiction de propriété de matériau selon la revendication 2, incluant la base de données de matériau (112) dans laquelle sont stockés les éléments suivants :
les premières données de tâche incluant une pluralité d'enregistrements, chacun incluant une composition de matériau, une première condition expérimentale et une première propriété de matériau ; et
les secondes données de tâche incluant une pluralité d'enregistrements, chacun stockant une composition de matériau et une seconde condition expérimentale définie différente de la première condition expérimentale.

4. Système de prédiction de propriété de matériau selon la revendication 2, incluant la base de données de matériau (112) dans laquelle sont stockés les éléments suivants :
les premières données de tâche incluant une pluralité d'enregistrements, chacun incluant une composition de matériau, une première condition expérimentale et une première propriété de matériau ; et
les secondes données de tâche incluant une pluralité d'enregistrements, chacun stockant une composition de matériau et une seconde propriété de matériau définie différente de la première propriété de matériau.

5. Système de prédiction de propriété de matériau selon la revendication 2, pourvu d'une base de données de modèle prédictif de propriété de matériau (114) stockant au moins l'un du premier modèle prédictif et du second modèle prédictif.

6. Système de prédiction de propriété de matériau selon la revendication 5, dans lequel le second modèle prédictif est géré en relation avec les secondes données de tâche.

7. Système de prédiction de propriété de matériau selon la revendication 1, dans lequel le premier modèle prédictif est configuré en utilisant une forêt aléatoire.

8. Procédé de prédiction de propriété de matériau qui est un procédé de prédiction de propriétés de matériau par un dispositif de traitement d'informations incluant un dispositif d'entrée, un dispositif de stockage et un processeur,
dans lequel lors de la génération d'un premier modèle prédictif pour prédire une première propriété de matériau à partir de premières données de tâche, les premières données de tâche incluant une pluralité d'enregistrements, chacun incluant une composition de matériau étant une formule structurelle du matériau, une condition expérimentale indiquant une condition lorsqu'une expérience associée à la première propriété de matériau a été menée, et la première propriété de matériau, le procédé exécute :
une première étape de préparation d'un second modèle prédictif qui consiste à prédire une seconde propriété de matériau définie différente de la première propriété de matériau en utilisant des compositions de matériau et la seconde propriété de matériau connue dans des secondes données de tâche différentes des premières données de tâche ;
une deuxième étape de prédiction de la seconde propriété de matériau en appliquant les premières données au second modèle prédictif ;
une troisième étape consistant à générer le premier modèle prédictif, en prenant la formule structurelle du matériau en tant que première variable explicative, la seconde propriété de matériau en tant que deuxième variable explicative, la condition expérimentale en tant que troisième variable explicative, et la première propriété de matériau en tant que variable objective, et une quatrième étape consistant à prédire la première propriété de matériau pour un enregistrement dans lequel la première propriété de matériau est inconnue en utilisant le premier modèle prédictif et les premières données.

9. Procédé de prédiction de propriété de matériau selon la revendication 8, utilisant une base de données de matériau sur une base par tâche,
dans lequel des premières données de tâche concernant une première tâche et des secondes données de tâche concernant une seconde tâche sont stockées dans la base de données de matériau.

10. Procédé de prédiction de propriété de matériau selon la revendication 9, dans lequel les premières données de tâche incluent en outre des premières informations sur des conditions de fabrication de matériau.

11. Procédé de prédiction de propriété de matériau selon la revendication 10, dans lequel les secondes données de tâche incluent en outre des secondes informations définies différentes des premières informations sur les conditions de fabrication de matériau.

12. Procédé de prédiction de propriété de matériau selon la revendication 8, dans lequel une forêt aléatoire est utilisée en tant que premier modèle prédictif.
